# EUROPÄISCHE PATENTANMELDUNG

(11) **EP 0 728 490 A2**
(43) Veröffentlichungstag der Anmeldung: **28.08.1996**
(21) Anmeldenummer: 96102297.7
(22) Anmeldetag: 15.02.1996
(51) Int. Cl.: A61M 1/32, A61M 1/36

(54) **Vorrichtung zur Behandlung von Blut**

(30) Priorität: 22.02.1995 DE 19506163
(71) Anmelder: Müller, Hans, 81547 München (DE)
(72) Erfinder: Müller, Hans, 81547 München (DE)
(74) Vertreter: Fleuchaus, Leo, Dipl.-Ing.

(57) **Zusammenfassung**

Eine Vorrichtung zur extrakorporalen Behandlung von Blut besteht aus einem Retransfusionsset (1), bei dem über einen Blutschlauch (10) und eine Steckspitze (7) Blut einer Vakuumflasche (2) zugeführt wird, in der über einen an die Steckspitze (7) angeschlossenen Gasschlauch (18) das Blut aufschäumbar ist, wobei das Gasmedium zum Aufschäumen des Blutes über einen Druckausgleichsbeutel (28) zugeführt wird, um in der Vakuumflasche das Restvakuum abzubauen und den Aufbau eines zu hohen Gasdrucks zu vermeiden. Im Blutschlauch (10) ist ferner eine Quarzglasküvette (22) zur UV-Bestrahlung des Blutes vorgesehen.

## Beschreibung

Die Erfindung betrifft eine Vorrichtung zur extrakorporalen Behandlung von einem menschlichen oder tierischen Körper entnommenen Blut nach dem Oberbegriff des Anspruchs 1.

Vorrichtungen zur Behandlung von menschlichem oder tierischem Blut mit "Sauerstoff, aktiviertem Sauerstoff oder mit sogenanntem Singulet-Sauerstoff" sind allgemein bekannt. Die sauerstoffhaltigen Behandlungsmedien werden nachfolgend mit dem Begriff "Gasbehandlung" bezeichnet. Bei dieser Behandlung wird das abgenommene Blut in einer Vakuumflasche oder einem Kunststoffbeutel der Gasbehandlung ausgesetzt und anschließend wieder in das menschliche oder tierische Blutgefäß retransfundiert. Eine solchen Vorrichtung geht aus den DE-OS 4238884 und 4340730 hervor und dient der Sauerstoffanreichung im Blut. Eine Einrichtung zur Gasbehandlung von Blut mittels der hämatogenen Oxidationstherapie (HOT) ist durch die DE-OS 3521802 bekannt, wobei das Blut bei der Entnahme in ein Auffanggefäß abgepumpt wird, in welches anschließend durch Austausch eine Vorrichtung zur HOT-Behandlung eingesetzt wird. Durch diese Offenbarung ist es bekannt, das Blut im Auffanggefäß durch Zufuhr von Sauerstoff aufzuschäumen und mit Hilfe des Überdrucks durch die Sauerstoffzufuhr durch eine Quarzglasküvette in ein weiteres Auffanggefäß zu drücken, wobei das aufgeschäumte Blut einer
UV-Bestrahlung ausgesetzt wird. Aus dem weiteren Auffanggerät wird das Blut anschließend wieder retransfundiert.

In der DE-OS 4410411 wurde eine Vorrichtung zur extrakorporalen Behandlung von Blut vorgeschlagen, bei der anstelle einer Vakuumflasche ein flexibler Blutbeutel Verwendung findet, der eine sehr einfache Handhabung der HOT-Behandlung ermöglicht und ein komplett montiertes Retransfusionsset zur Verfügung stellt, das in seiner Gesamtheit in einer verhältnismäßig kleinen Packung steril verpackt und zum Einsatz gebracht werden kann. Da bei der Blutabnahme kein Unterdruck Verwendung findet, wird für die Blutabnahme verhältnismäßig viel Zeit benötigt.

Deshalb wird - nach wie vor - häufig zur Verwendung einer Vakuumflasche gegriffen, um die Blutabnahme mit Hilfe des Vakuums zu beschleunigen.

Ein Problem bei der Gasbehandlung des Blutes ergibt sich dadurch, daß das Begasungsmedium aus einer unter hohen Druck stehenden Vorratsflasche über ein Reduzierventil zugeführt wird. Die üblicherweise verwendeten Reduzierventile können in der Regel nur auf einen verhältnismäßig hohen Abströmdruck eingestellt werden, so daß sich der Aufbau eines Überdrucks in der Vakuumflasche bzw. im Blutbeutel bei der Gasbehandlung nur schwer vermeiden läßt.

Der Erfindung liegt deshalb die Aufgabe zugrunde, Maßnahmen zu schaffen, mit denen die Begasung des Blutes mit der gewünschten Gasmenge in einfacher Weise möglich ist, ohne daß ein Überdruck in der Vakuumflasche oder dem Blutbeutel entsteht.

Diese Aufgabe wird durch die Maßnahmen des Anspruchs 1 gelöst. Weitere Ausgestaltungen der Erfindung sind Gegenstand von Unteransprüchen.

Durch die Verwendung eines Druckausgleichsbeutels in der Zuleitung des Gasmediums läßt sich dieser, bevor das Gasmedium dem Blut zugeführt wird, mit einer vorgegebenen Luftmenge aufblasen, die für die Begasung des Blutes ohne den Aufbau eines nennenswerten Überdrucks in der Vakuumflasche oder im Blutbeutel ausreicht. Wenn größere Luftmengen benötigt werden, ist es auch möglich, den Druckausgleichsbeutel mehrfach aufzublasen.

Durch die Maßnahmen der Erfindung ist es in einfacher Weise möglich, einen für das Retransfundieren des Blutes unerwünschten Druckaufbau in der Vakuumflasche bzw. dem Blutbeutel zu vermeiden.

Gemäß einer weiteren Ausgestaltung der Erfindung ist vorgesehen, daß der elastische Druckausgleichsbeutel gefaltet ist.

Durch eine spezielle Ausgestaltung der Steckspitze kann das für die Gasbehandlung zugeführte Gasmedium durch einen Diffusionskörper in das Blut eingeleitet werden.

Die Vorteile und Merkmale der Erfindung ergeben sich auch aus der nachfolgenden Beschreibung eines Ausführungsbeispieles in Verbindung mit der Zeichnung; es zeigen:
- Fig. 1: ein Retransfusionsset gemäß der Erfindung;
- Fig. 2: eine weitere Ausführungsform des Retransfusionssets;
- Fig. 3: ein Schauglas mit einer Steckspitze mit Diffusionskörper;
- Fig. 4: eine Steckspitze.

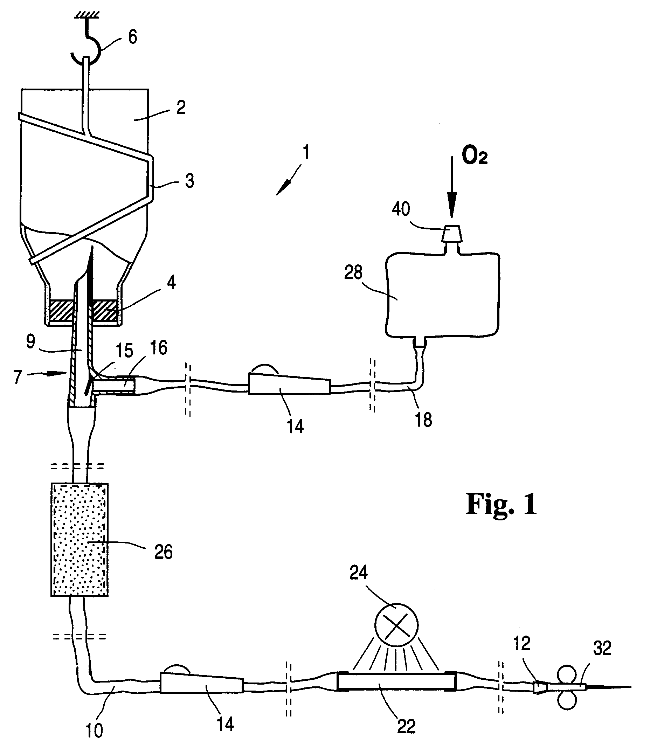

In Fig. 1 ist ein Retransfusionsset 1 für die Gas-Behandlung von Blut dargestellt, das mit Ausnahme der Vakuumflasche 2 als fertigmontiertes Besteck in steril versiegelten Blisterpackungen zur Verfügung steht. Die Vorrichtung besteht aus der Vakuumflasche 2, welche mit Hilfe einer Aufhängung 3 an einem Haken 6 aufgehängt werden kann. Durch den Gummistopfen 4 der Vakuumflasche ist eine Steckspitze 7 mit Kanüle 9 eingestochen. Die kanüle 9 steht mit einem Blutfilter 26 sowie einen Gasanschluß 16 in Verbindung, der über ein Gasventil 15 in die Kanüle 9 mündet.

Hinter dem Blutfilter 26 führt in herkömmlicher Weise ein Blutschlauch 10 zu einer Quarzglasküvette 22 und von dort aus über ein Kupplungsstück 12 an eine Flügelkanüle 32. Zwischen der Quarzgasküvette 22 und dem Blutfilter 26 ist auf dem Blutschlauch 10 eine Rollklemme 14 montiert, mit welcher der Blutschlauch abgeklemmt werden kann. Die Quarzglasküvette 22 liegt im Strahlungsbereich einer UV-Lichtquelle 24.

An dem Gasanschluß 16 ist ein Gasschlauch 18 angeschlossen auf dem sich ebenfalls eine Rollklemme 14 zum Abquetschen des Gasschlauches befindet. Der Gasschlauch ist an dem dem Schauglas 8 gegenüberliegenden Ende an einen Druckausgleichsbeutel 28 angeschlossen, der seinerseits mit einer Kupplung 40 für den Anschluß einer Gasquelle versehen ist. Diese Gasquelle besteht in üblicher Weise aus einem unter hohen Druck stehenden Vorratsbehälter, von dem das Gasmedium über ein Reduzierventil dem Druckausgleichsbeutel zugeführt werden kann. Bei derartigen Reduzierventilen liegt der minimale Abströmdruck in der Regel zu hoch, so daß ein direktes Zuführen des Gasmediums in die Vakuumflasche in dieser einen hohen Druck aufbaut, der nur über ein zusätzliches Druckausgleichsventil vor dem Retransfundieren des Blutes abgebaut werden kann.

Durch die Verwendung des Druckausgleichsbeutels 28 läßt sich dieser unerwünschte hohe Druckaufbau in der Vakuumflasche vermeiden.

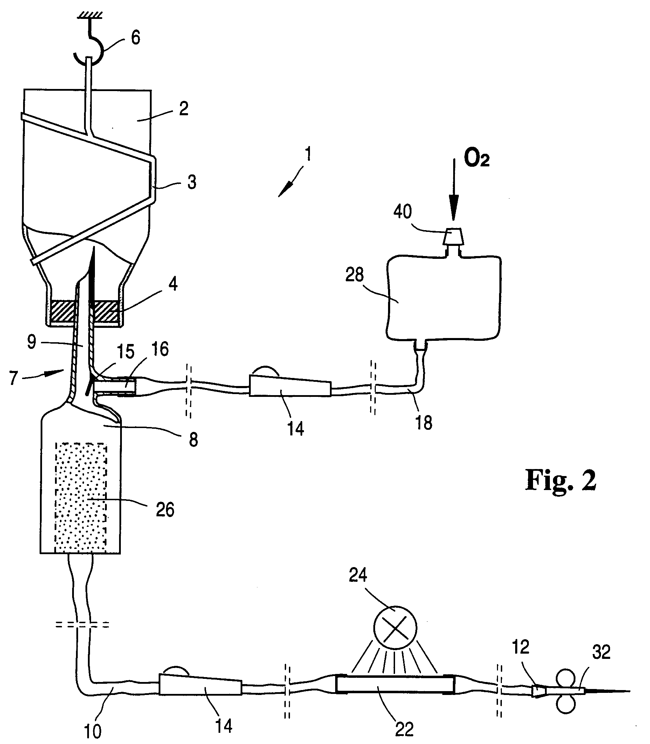

In Fig. 2 ist eine Ausführungsform der Erfindung dargestellt, bei der das Blutfilter 26 und die Steckspitze zu einer als Schauglas 8 ausgebildeten Einheit zusammengefaßt sind.

Eine Blutbehandlung mit Hilfe des Retransfusionssets läßt sich in der Regel wie folgt ausführen:

Zu Beginn der Behandlung wird das einteilige, aus mehreren Komponente bestehende Retransfusionsset einer sterilen Verpackung entnommen und die Kanüle 9 der Steckspitze 7 durch den Gummistopfen 4 der Vakuumflasche 2 gestochen. Ferner wird die Kupplung 40 am Druckausgangsbeutel 28 an eine unter höheren Druck stehende Gasquelle angeschlossen, die beispielsweise eine Sauerstoffflasche mit Reduzierventil sein kann. Derartige Reduzierventile lassen sich auf einen minimalen Abströmdruck in der Größenordnung von etwa 5 bar einstellen, ein Druck, der für die einfache Handhabung des Retransfusionssets zu hoch ist.

Zum Abnehmen des Blutes wird mit der Flügelkanüle 32 ein Blutgefäß punktiert, und das Blut über den Blutschlauch 10, die geöffnete Rollklemme 14 und das Blutfilter 26 in die Vakuumflasche 2 gesaugt.

Zu Behandlung mit dem Gasmedium wird die Rollklemme 14 am Blutschlauch 10 geschlossen und die Rollklemme 14 am Gasschlauch 18 geöffnet. Damit kann die im Druckausgleichsbeutel 28 vorgegebene und gegenüber dem atmosphärischen Normaldruck etwas unter Überdruck stehende Luftmenge über den Gasanschluß 16 und das Gasventil 15 sowie die Kanüle 9 in die Vakuumflasche einströmen, wobei das Blut aufgeschäumt wird und sich das Vakuum in der Vakuumflasche abbaut. Wenn die im Druckausgleichsbeutel 28 vorhandene Luftmenge etwa der Menge entspricht, die für den Vakuumabbau benötigt wird, ergibt sich während der Gasbehandlung des Blutes ein Druckaufbau auf Normaldruck, so daß nunmehr für das Retransfundieren das Blut in der Vakuumflasche nahezu drucklos zur Verfügung steht. Zum Retransfundieren wird die Rollklemme 14 am Blutschlauch 10 geöffnet. Gleichzeitig bleibt auch die Rollklemme 14 am Gasschlauch 18 geöffnet, damit über den Druckausgleichsbeutel 28 ein Druckausgleich beim Retransfundieren erfolgt. Dazu dient entweder eine Restgasmenge im Druckausgleichsbeutel oder aber über das offene Kupplungsstück 40 zuströmende Luft. Beim Retransfundieren wird das durch die Quarzglasküvette 22 über die Flügelkanüle in das Gefäß zurückfließende und mit Sauerstoff aufgeschäumte Blut mit Hilfe der UV-Quelle 24 bestrahlt. Anstelle einer UV-Quelle kann auch eine Breitbandlichtquelle Verwendung finden.

Wenn zum Retransfundieren die Vakuumflasche 2 an einem Haken 6 aufgehängt ist, kann durch die Einstellung der Höhe der Aufhängung der Rückfluß des Blutes in das Blutgefäß gegen den in dem Gefäß anstehenden Blutdruck eingestellt werden. Die Vorrichtung bietet die Möglichkeit, die UV-Bestrahlung des Blutes sowohl bei der Abnahme des Blutes als auch beim Retransfundieren vorzunehmen. Dadurch läßt sich der Zeitaufwand für die hämatogene Oxidationstherapie verringern.

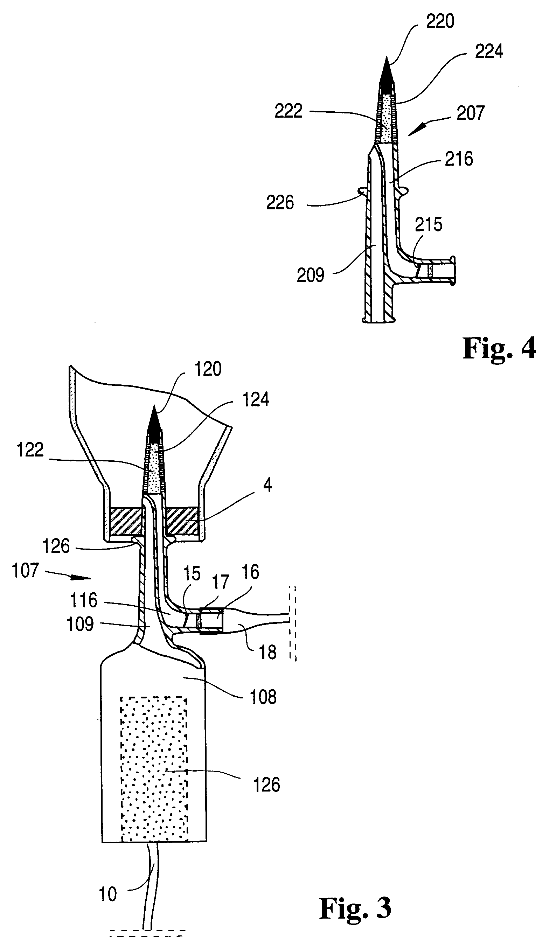

Eine besonders vorteilhafte Ausführungsform des Schauglases 118 ist in Fig. 3 dargestellt. Dieses Schauglas unterscheidet sich von einem herkömmlichen Schauglas dadurch, daß der Gasanschluß 16 in der Steckspitze 107 mit einer separaten Leitung 116 zur Spitze des Schauglases geführt wird. In dieser Spitze des Schauglases ist hinter einer eingegossenen Stahlspitze 120 ein Diffusionskörper 122 angeordnet und ferner ist der Bereich der Wandung des mit der Stahlspitze versehenen Dorns siebartig mit Kleinen Löchern 124 versehen, so daß das über den Gasschlauch 18 zugeführte Gasmedium in feinster Verteilung in die Vakuumflasche 2 eingeleitet wird. Diese Kleinen feinverteilten Gasbläschen erhöhen den Wirkungsgrad der Gasbehandlung ganz wesentlich.

Um sicherzustellen, daß beim Einstecken des Dorns in den Gummistopfen 4 die Austritts- und Eintrittsöffnung für das Blut an der Kanüle 109 sicher im Innern der Vakuumflasche 2 zu liegen kommt, ist an dem Dorn ein Wulst 126 angebracht der beim Einstecken auf der Oberfläche des Gummistopfens zur Anlage kommen soll.

Schließlich ist in der Gasleitung 16 vorzugsweise im Bereich vor dem Gasventil 15 ein Bakterienfilter 17 vorgesehen.

In Fig. 4 ist eine Steckspitze 207 gezeigt, die sich von der Ausführungsform nach Fig. 3 in ihrem Aufbau nicht wesentlich unterscheidet, jedoch nicht einstückig an ein Schauglas angeformt ist. Eine derartige Steckspitze ist insbesondere für die Ausführungsform der Erfindung nach Fig. 1 geeignet.

Durch die Integration des Diffusionskörper in den Dorn des Schauglases 8 bzw. 108 ergibt sich eine viel zweckmäßigere Ausgestaltung des Retransfusionssets, wobei gleichzeitig die Kosten gegenüber herkömmlichen Diffusionsvorrichtungen verringert werden können.

Der wesentliche Vorteil der Erfindung wird darin gesehen, daß durch den gezielten und geregelten Druckaufbau bei der Zuführung des Gasmediums zum Aufschäumen des Blutes mit großer Sicherheit in der Vakuumflasche das Entstehen eines zu hohen Drucks vermieden wird. Der Druckausgleichsbeutel macht das dosierte Zuführen des Gasmediums in einer vorgegebenen Menge leicht möglich, da in dem Druckausgleichsbeutel ein zur Begasung des Blutes ausreichender Gasvorrat geschaffen werden kann, der beim Aufschäumen des Blutes in der Vakuumflasche keinen überhöhten Überdruck entstehen läßt. Auf diese Wiese läßt sich über den Druckausgleichsbeutel ein Restvakuum nach der Blutentnahme leicht abbauen und das zum Aufschäumen benötigte Gasmedium in der gewünschten Menge fein verteilt zuführen, ohne daß ein übermäßiger Druckaufbau in der Vakuumflasche entsteht.

## Patentansprüche

1. Vorrichtung zur extrakorporalen Behandlung von einem menschlichen oder tierischen Körper entnommenen Blut mit einer in eine Vakuumflasche (2) einsteckbaren Steckspitze, einem mit der Steckspitze über ein Blutfilter (26) und eine Durchflußregeleinrichtung {Rollklemme (14)} kommunizierenden und mit einem Kupplungsstück (12) versehene Blutschlauch (10) sowie mit einem ebenfalls mit der Vakuumflasche (2) kommunizierenden Gasschlauch (18),
**dadurch gekennzeichnet,**
daß im Blutschlauch (10) zwischen dem Kupplungsstück (12) und dem Blutfilter (26) eine Quarzglasküvette (22) angeordnet ist;
daß ein unter Normaldruck stehender elastischer Druckausgleichsbeutel (28) im Gasschlauch gasseitig hinter der Druchflußregeleinrichtung (14) angeordnet ist;
und daß der Gasschlauch (18) im Bereich der Steckspitze über ein Rückschlagventil (15) an den Blutschlauch (10) angeschlossen ist.

2. Vorrichtung nach Anspruch 1
**dadurch gekennzeichnet,**
daß der Druckausgleichsbeutel (28) aus einem gefalteten unter Normaldruck stehenden Kunststoffbeutel besteht.

3. Vorrichtung nach Anspruch 1 oder 2
**dadurch gekennzeichnet,**
daß das Blutfilter (26) in einem mit der Steckspitze verbundenen Schauglas (8) angeordnet ist.

4. Vorrichtung nach einem der Ansprüche 1bis 3
**dadurch gekennzeichnet,**
daß die Vorrichtung (1) aus einem fest miteinander verbundenen und als eine Einheit verwendungsfertig vorliegenden Teil besteht.

5. Vorrichtung nach einem der Ansprüche 1 bis 4
**dadurch gekennzeichnet,**
daß die Steckspitze mit einer an die Gasleitung (18) anschließbaren Kanüle (216) und einer Kanüle (209) für die Blutführung versehen ist;
daß in der Spitze der Steckspitze hinter einer eingegossenen Stahlspitze (220) ein Diffusionskörper (222) angeordnet ist, wobei der Bereich der Wandlung um den Diffusionskörper siebartig mit Kleinen Löchern versehen ist;
und daß die Kanüle (209) hinter dem Diffusionskörper aus dem Schaft der Steckspitze austritt.

6. Vorrichtung nach Anspruch 5
**dadurch gekennzeichnet,**
daß die Steckspitze in ihrem hinteren Teil mit einem Wulst (226) versehen ist, der in einem vorgegebenen Abstand zur Mündung der Kanüle (209) im Schaft um die Steckspitze derart verläuft, daß die Mündung der Kanüle (209) beim Einstecken der Steckspitze in einen Gummistopfen (49) im Innern der Vakuumflasche und der Wulst (216) außen an dem Gummistopfen zur Anlage kommt.
